# EUROPÄISCHE PATENTSCHRIFT

(11) **EP 1 276 439 B1**
(45) Veröffentlichungstag und Bekanntmachung des Hinweises auf die Patenterteilung: **24.03.2010**
(21) Anmeldenummer: 01945061.8
(22) Anmeldetag: 27.04.2001
(51) Int. Cl.: A61F 2/24, A61L 27/00

(54) **INDIVIDUELLE VENENKLAPPENPROTHESE**
INDIVIDUAL VENOUS VALVE PROSTHESIS
PROTHESE INDIVIDUELLE DE VALVULE DU SINUS VEINEUX

(30) Priorität: 27.04.2000 DE 10020540
(43) Veröffentlichungstag der Anmeldung: 22.01.2003
(73) Patentinhaber: Corlife GbR, 30625 Hannover (DE)
(72) Erfinder: Corlife GbR, 30625 Hannover (DE)
(74) Vertreter: Grünecker, Kinkeldey, Stockmair & Schwanhäusser Anwaltssozietät
(86) Internationale Anmeldenummer: PCT/EP2001/004796
(87) Internationale Veröffentlichungsnummer: WO 2001/080782

(56) Entgegenhaltungen:
- WO-A-00/64569
- WO-A-01/28459
- WO-A-96/31157
- WO-A-98/49972
- US-A- 5 733 337
- US-A- 5 863 296

## Beschreibung

Die Erfindung betrifft die Verwendung einer empfängerspezifisch transformierten synthetischen Matrix für die Herstellung einer individuellen Venenklappenprothese.

Venenleiden nehmen unter den Zivilisationskrankheiten einen wesentlichen Platz ein. Jährlich erkranken zahlreiche Menschen, vorwiegend in den sogenannten Industrienationen an Veneninsuffizienz. Mangelnde Bewegung trägt ebenso zur zunehmenden Verbreitung dieses Krankheitsbildes bei, wie Emährungsfehler und Übergewicht. Die chronische Veneninsuffizienz ist ein Problem von erheblichem gesundheitspolitischen Interesse, da ein beträchtlicher Teil der erwachsenen Bevölkerung betroffen ist und längere Krankenhausaufenthalte, im Einzelfall Arbeitsunfähigkeit, die letztliche Folge sein können. Auch die Gefahr für Lungenembolien als Folge von Thrombosen stellt ein erhebliches Risiko dar. Durch konservative Behandlung ist der chronischen Veneninsuffizienz kaum beizukommen, behandelt wird mit Stützstrümpfen und -verbänden. Im fortgeschrittenen Zustand werden die Venenklappen völlig zerstört, d.h. es treten aufgelöste, andererseits auch verdickte Bereiche in den Venenklappen auf. Klinische Folge sind schmerzhafte, ästhetisch störende und äußerst langwierig zu behandelnde sogenannte offene Beine oder Ulcus cruris. In diesem Stadium ist nur noch eine chirurgische Behandlung möglich. Ein solcher Eingriff fällt in das Gebiet der Gefäßchirurgie; hierbei versucht der Gefäßchirurg die funktionsuntüchtige Venenklappe zu rekonstruieren. Es wurden verschiedene Verfahren erprobt, zu denen die Venenrekonstruktion nach Rutherford und die Valvuloplastie nach Kistner gehören. Aufgrund unzureichender klinischer Erfolge hat sich allerdings keines der Verfahren bisher durchgesetzt.

Der Nachteil der verschiedenen chirurgischen Rekonstruktionsverfahren besteht darin, dass die Klappe nicht vollständig in den Ursprungszustand zurückversetzt werden kann, so dass ein Risiko für weitere Verschlechterungen, insbesondere, Ulcus cruris, bleibt.

Ein Problem bei der chirurgischen Behandlung besteht auch darin, dass die Rekonstruktion direkt am Patienten erfolgen muss.

Aufwendige Rekonstruktionsverfahren bringen daher den Nachteil längerer Operationszeiten und die damit verbundenen Risiken mit sich. Nachteilig ist auch, dass die Venenklappe nach der Operation sofort wieder vollständig belastbar sein muss, das Operationsgebiet also in keiner Weise geschont werden kann. Operationsverletzungen, Narben oder selbst kleinere Gerinnsel an der Venenklappe führen zu einem relativ hohen Thromboserisiko, das den Erfolg dieser chirurgischen Verfahren gleich wieder in Frage stellt.

WO 98/49972 offenbart eine Einrichtung zur Behandlung verschiedener Teile eines prothetischen Produkts, das Gefäßgewebe, z. B. eine Herzklappe umfassen kann. Das Gefäßgewebe kann azellularisiertes Gewebe sein.

US 5 863 296 offenbart bioprothetische Xenotransplantate, z.B. Herzklappenimplantate, deren endogene Zellen vor der Weiterbehandlung abgetötet werden.

In der WO 96/31157 wird die Verwendung einer natürlichen xenogenen Matrix zur Herstellung einer Venenklappenprothese beschrieben.

WO 00/64569 offenbart die Herstellung einer Venenklappenprothese (siehe Seite 5, Zeilen 23 - 26 im Zusammenhang mit Seite 16, Zeilen 38 - 40) aus einer azellularisierten Matrix (Seite 17, Zeilen 24 - 28), wobei die azellularisierte Matrix mit autologen Zellen besiedelt wird

WO 01/28459 offenbart vergleichbare Merkmale wie die WO 00/64569, wobei als Matrix ein Venenklappenrahmen aus einem synthetischen, biologisch abbaubaren Material zur Verfügung gestellt wird.

In einer Ausführungsform umfasst die Prothese einen röhrenförmigen Fluidkanal mit einem Stent, wobei die Ventilklappen an der inneren Oberfläche des Stents befestigt sind.

WO 00/64569 und WO 01/28459 fallen unter Art. 54(3) EPÜ.

Die Aufgabe der Erfindung besteht daher darin, neue Möglichkeiten für die Bekämpfung der Veneninsuffizienz zu eröffnen.

Zur Lösung dieser Aufgabe ist erfindungsgemäß die Verwendung einer empfängerspezifisch transformierten azellularisierten Matrix für die Herstellung einer individuellen Venenklappenprothese vorgesehen.

Die vorliegende Erfindung stellt ein Verfahren zur Herstellung einer individuellen Venenklappenprothese gemäß Anspruch 1 zur Verfügung.

Zwar sind andere Gefäßprothesen prinzipiell bereits bekannt, beispielsweise werden seit Jahren Herzklappenprothesen relativ erfolgreich eingesetzt. Für Venenklappen sind die bislang für Herzklappen durchgesetzten und kommerziell verbreiteten Klappenmaterialien jedoch nicht geeignet. Es hat sich gezeigt, dass die Thrombosegefahr im Bereich der kleiner dimensionierten Venenklappen unter den dort herrschenden andersartigen Strömungsbedingungen (geringer Fluß, geringe Druckgradienten) zu hoch ist. Dem Gefäßchirurg war daher bislang keine Möglichkeit gegeben, das Krankheitsbild anders als mit den oben beschriebenen unzulänglichen Verfahren zu behandeln.

Überraschenderweise wurde nun gefunden, dass eine empfängerspezifisch transformierte azellularisierte Matrix für die Herstellung einer individuellen Venenklappenprothese geeignet ist.

Ein großer Vorteil der Erfindung liegt darin, dass die fertige, "heile" Venenklappenprothese insgesamt in einem kürzeren Operationsschritt eingesetzt werden kann und dabei die defekte Klappe ersetzt. Die Anschlüsse liegen an glatten Schnitten, die vergleichsweise unkompliziert zu verbinden sind und von einem Thromboserisiko in der Klappe selbst wegführen.

Da die individuelle Venenklappenprothese an den ausgesuchten Empfänger speziell angepaßt ist, kann das Thromboserisiko minimal gehalten werden.

Unter einer "empfängerspezifischen Transformation" wird dabei vorzugsweise eine Besiedlung der ausgewählten Matrix mit empfängerverträglichen Zellen, insbesondere mit autologen Zellen des Protheseempfängers verstanden.

Die unten noch näher beschriebene Matrix wird soweit mit empfängerverträglichen Zellen besiedelt, dass die Thrombogenizität des Fremdkörpers "Venenklappenprothese" hinreichend unterdrückt wird. Auch die Art der aufgesiedelten Zellen hat auf die Thrombogenizität einen Einfluss.

Besonders geeignet ist eine Besiedlung mit Fibroblasten und Endothelzellen sowie auch ggf. mit Myofibroblasten.

Die Matrix, die für die empfängerspezifische Transformation verwendet wird, eine synthetische Matrix ist, beispielsweise aus einem biopolymeren Material, einem für Prothesen üblichen polymeren Material und insbesondere einem biologisch abbaubaren polymeren Material. Ein geeignetes Material wäre z.B. ein Lactid-enthaltendes Polymer, vorzugsweise ein Copolymer aus Lactid und einer Glykol-Verbindung, weiter vorzugsweise mehrfach geschichtetes Polydioxanon.

In einer Ausführungsform nicht gemäß der Erfindung, kann die Matrix auch eine für den Empfänger xenogene oder allogene Matrix sein, aus der oder von deren Oberfläche vor der empfängerspezifischen Transformation im wesentlichen alle natürlichen Zellen entfernt wurden.

Vorzugsweise kann die für die empfängerspezifische Transformation verwendete Grundmatrix eine natürliche Venenklappe sein. Die Azellularisierung solcher xenogener oder allogener Venenklappen kann in an sich bekannter Weise geschehen, beispielsweise durch enzymatische Ablösung der Zellen, z.B. mit Trypsin, oder durch Ablösen, und/oder Abtöten der Zellen mit chemischen und/oder mechanischen Mitteln.

Gemäß der Erfindung wird ein empfängerspezifisch transformiertes Matrixmaterial für die Konstruktion einer Venenklappenprothese verwendet. Hierbei kann so vorgegangen werden, dass die Venenklappenprothese aus mehreren Matrixbestandteilen zusammengesetzt ist. Die ausgewählten Materialien werden vor der Konstruktion der Venenklappenprothese vorbesiedelt und können ggf. nach der Konstruktion in einem weiteren Verfahrensschritt mit einem nicht thrombogenen Material an der Oberfläche beschichtet oder zusätzlich weiter mit empfängerspezifischen Zellen besiedelt werden.

Die Venenklappenprothese umfasst dabei vorzugsweise wenigstens ein Klappensegel.

Um einen besseren Anschluss an die Vene des Empfängers zu gewährleisten, die Venenklappenprothese umfasst ein Venenstück einer gewissen Gesamtlänge, wobei vorgesehen ist, dass die Venenklappe sich in einem Venenstück befindet, dessen Länge oberhalb und unterhalb des Klappenbereiches jeweils wenigstens einmal dem Durchmesser der Vene bzw. dem Klappenquerschnitt entspricht.

Ein großer Vorteil der Erfindung liegt darin, dass die in der angegebenen Weise prozessierte und ggf. für den Empfänger speziell neu konstruierte Venenklappe in ggf. mehreren Vertahrensschritten so vorbereitet werden kann, dass die Thrombosegefahr für den zugehörigen ausgewählten Empfänger möglichst gering bleibt.

Vorteilhaft ist auch, dass die Funktion der Venenklappenprothese wenigstens in vitro getestet werden kann, indem die Venenklappe in eine entsprechende Vorrichtung eingespannt und pulsierend mit einem Kulturmedium oder auch einfacher (kristalloider) Lösung perfundiert wird.

## Patentansprüche

1. Verfahren zur Herstellung einer individuellen Venenklappenprothese, mit den Schritten:
Bereitstellen einer synthetischen Matrix,
Besiedeln der Matrix mit autologen Zellen des Prothesenempfängers,
Herstellen der Venenklappenprothese unter Verwendung der besiedelten Matrix, so dass die Venenklappenprothese ein Venenstück einer Länge von wenigstens einmal dem Durchmesser des Klappenquerschnitts oberhalb und unterhalb der Klappe umfasst, wobei bei der hergestellten Venenklappenprothese keine Ventilklappen an der inneren Oberfläche eines Stents festgemacht sind.

2. Verfahren nach Anspruch 1, wobei die synthetische Matrix aus einem Biopolymer, einem Polymer, oder einem biologisch abbaubaren Polymer bereitgestellt wird.

3. Verfahren nach Anspruch 1 oder 2, wobei die Venenklappenprothese wenigstens ein Klappensegel umfasst.

## Claims

1. Method for producing an individual venous valve prosthesis, comprising the steps of:
preparing a synthetic matrix,
populating the matrix with autologous cells of the prosthesis recipient,
producing the venous valve prosthesis using the populated matrix, in such a way that the venous valve prosthesis comprises a vein portion of a length at least one times the diameter of the valve cross-section above and below the valve, wherein no valve leafs are attached to the internal surface of a stent in the venous valve prosthesis produced.

2. Method according to claim 1, wherein the synthetic matrix is prepared from a biopolymer, a polymer or a biodegradable polymer.

3. Method according to either claim 1 or claim 2, wherein the venous valve prosthesis comprises at least one valve cusp.

## Revendications

1. Procédé pour fabriquer une prothèse individuelle de valve veineuse, avec les étapes qui consistent :
à préparer une matrice synthétique,
à implanter dans la matrice des cellules autologues du receveur de prothèse,
à fabriquer la prothèse de valve veineuse en utilisant la matrice avec lesdites cellules implantées, de telle sorte que la prothèse de valve veineuse comprenne une partie de veine d'une longueur égale à au moins une fois le diamètre de la section transversale de la valvule au-dessus et au-dessous de celle-ci, étant précisé qu'avec la prothèse de valve veineuse fabriquée, aucune valvule n'est fixée à la surface intérieure d'un stent.

2. Procédé selon la revendication 1, la matrice synthétique étant préparée à partir d'un biopolymère, d'un polymère ou d'un polymère biodégradable.

3. Procédé selon la revendication 1 ou 2, la prothèse de valve veineuse comprenant au moins une valvule.
